Europäisches Patentamt

European Patent Office

Office européen des brevets

(11) Numéro de publication: **0 275 748 B1**

# FASCICULE DE BREVET EUROPEEN

(45) Date de publication de fascicule du brevet: **19.08.92**

(51) Int. Cl.5: **C07K 7/06**, C07K 5/10, C12Q 1/37

(21) Numéro de dépôt: **87402845.9**

(22) Date de dépôt: **14.12.87**

Le dossier contient des informations techniques présentées postérieurement au dépôt de la demande et ne figurant pas dans le présent fascicule.

(54) **Nouveaux dérivés peptidiques et leur application notamment en thérapeutique.**

(30) Priorité: **15.12.86 FR 8617507**
**25.06.87 FR 8708986**

(43) Date de publication de la demande:
**27.07.88 Bulletin 88/30**

(45) Mention de la délivrance du brevet:
**19.08.92 Bulletin 92/34**

(84) Etats contractants désignés:
**AT BE CH DE ES FR GB GR IT LI LU NL SE**

(56) Documents cités:
**EP-A- 0 164 654**

**CHEMICAL ABSTRACTS, vol. 102, 1985, page 428, résumé no. 21570a, Columbus, Ohio, US; J.C. BOUCAUT et al.: "Biologically active synthetic peptides as probes of embryonic development: a competitive peptide inhibitor of fibronectin function inhibits gastrulation in amphibian embryos and neural crest cell migration in avian embryos", & J. CELL. BIOL. 1984, 99(5), 1822-30**

**CHEMICAL ABSTRACTS, vol. 107, 1987, pages 478, résumé no. 173261s, Columbus,**

**Ohio, US; E.F. PLOW et al.: "Arginyl-glycyl-aspartic acid sequences and fibrinogen binding to platelets", & BLOOD 1987, 70(1), 110-15**

(73) Titulaire: **INSTITUT NATIONAL DE LA SANTE ET DE LA RECHERCHE MEDICALE (INSERM) 101, rue de Tolbiac F-75654 Paris Cédex 13(FR)**

Titulaire: **LABORATOIRE L. LAFON 19 Avenue du Professeur Cadiot F-94701 Maisons Alfort(FR)**

(72) Inventeur: **Marguerie de Rotrou, Gérard Armand 5, Avenue Albert 1er de Belgique F-38000 Grenoble(FR)**

(74) Mandataire: **Le Guen, Gérard et al CABINET LAVOIX 2, place d'Estienne d'Orves F-75441 Paris Cédex 09(FR)**

Rank Xerox (UK) Business Services

## Description

La présente invention concerne de nouveaux dérivés peptidiques ayant une activité antiagrégante, leur procédé de préparation et leurs applications en thérapeutique et comme agents de diagnostic.

De façon plus précise, elle concerne des analogues peptidiques d'une séquence du fibrinogène utilisables notamment comme antagonistes du fibrinogène vis à vis des plaquettes sanguines.

On sait qu'au cours de l'hémostase les plaquettes sanguines adhèrent au sous-endothélium du vaisseau lésé, secrètent leur contenu granulaire après stimulation et agrègent les unes aux autres pour former un thrombus plaquettaire. L'agrégation dépend des contacts qui s'établissent entre membranes de plaquettes adjacentes. Cette réaction est nécessaire pour l'arrêt d'un saignement. Elle a cependant de nombreuses déviations pathologiques, notamment dans les cas de thromboses veineuses ou artérielles, au cours du développement d'une plaque d'athérome, ou de la formation de microthrombi qui peuvent obstruer la microcirculation périphérique ou cérébrale. Le contrôle et la régulation de l'agrégation plaquettaire sont donc un objectif majeur dans la prévention de la thrombose et de l'athérosclérose et de nombreuses études sont consacrées à la recherche et au développement de molécules aux propriétés antiagrégantes.

Dans ce phénomène, le fibrinogène a un rôle important. Ainsi dans le plasma de malades atteints d'afibrinogènémie congénitale, l'agrégation plaquettaire est fortement diminuée ou absente et ce défaut est corrigé par l'injection de fibrinogène. De même en l'absence de fibrinogène, des plaquettes lavées n'agrègent pas à l'ADP ou à l'épinéphrine. La présence de fibrinogène est donc une nécessité pour le développement normal d'un thrombus plaquettaire. La participation du fibrinogène à l'agrégation est due à l'induction d'un récepteur spécifique pour cette protéine sur la membrane de la plaquette activée. Tous les stimuli physiologiques de la plaquette induisent une classe unique de récepteur et l'interaction du fibrinogène avec ce récepteur régule l'agrégation plaquettaire. Il existe donc un mécanisme de l'agrégation plaquettaire, commun à tous les inducteurs, qui dépend de l'interaction entre le fibrogène et son récepteur. L'importance physiologique de cette voie de l'agrégation plaquettaire dépendante du fibrinogène est attestée par l'étude des thrombasthénies de Glanzmann dont les plaquettes ne fixent pas le fibrinogène et n'agrègent pas en réponse à tous les stimuli physiologiques de la cellule.

En résumé, le récepteur du fibrinogène n'est pas exprimé sur la plaquette circulante, il est induit dès que la cellule est stimulée. Cette induction peut être dépendante ou indépendante de la réaction de sécrétion. Tous les stimuli expriment le même récepteur et l'interaction entre le fibrinogène et le récepteur conduit directement à l'agrégation. La dissociation du fibrinogène lié à la plaquette a pour conséquence la désagrégation des plaquettes.

Dans ce contexte il est clair que si l'interaction entre le fibrinogène et son récepteur plaquettaire pouvait être régulée, ceci constituerait un moyen pour contrôler l'agrégation in vitro et in vivo.

La présente invention vise précisément à fournir de nouveaux agents qui permettent d'inhiber, de réguler ou de mesurer sélectivement la voie de l'agrégation dépendante du fibrinogène.

Des séquences peptidiques issues de la molécule de fibrinogène ont déjà été identifiées comme inhibant la fixation de cette protéine sur les plaquettes et bloquant ainsi leur agrégation. Ainsi E. Plow et coll. (Proc. Natl. Acad. Sci. USA 82 8057, 1985) ont décrit l'activité de la séquence Arg-Gly-Asp-Ser (RGDS)* présente dans la chaîne alpha du fibrinogène.

La présente invention a pour objet des analogues peptidiques de l'extrémité C-terminale de la chaîne alpha du fibrinogène qui présentent un effet inhibiteur de la liaison fibrinogène-plaquette et de l'agrégation plaquettaire.

Ces dérivés peptidiques répondent à la formule générale suivante :

$$X^1 - X^2 - Gly - Asp - X^3 - X^4 \qquad (I)$$

dans laquelle

$X^1$ représente l'hydrogène, un groupe N-protecteur, un résidu d'acide aminé ou un résidu d'acide aminé N- protégé,

$X^2$ représente un résidu L-Arg ou D-Arg, L-Orn ou D-Orn, N-aminocarbonyl -L-Orn ou N-amino carbonyl-D-Orn, ou L-Lys ou D-Lys,

$X^3$ représente un résidu L-Trp, D-Trp, L-Leu, D-Leu, L-Ile, D-Ile, L-Phe, D-Phe ou un enchaînement de 2 ou 3 de ces résidus, et

$X^4$ représente un groupe $-OH, -NH_2, -OR^1$ avec $R^1$ représentant un radical alkyle en $C_1$ à $C_4$, $NHR^2$ avec

---

* La signification des symboles et abréviations utilisées est donnée en annexe.

$R^2$ représentant un radical alkyle en $C_1$ à $C_4$, ou un résidu d'acide aminé.

Dans les groupes $X^1$ et $X^4$ les résidus d'acides aminés susceptibles d'être utilisés sont notamment les radicaux D ou L-pyroglutamyle, L ou D-alanyle, glycyle, L ou D-prolyle, L ou D-valyle, L ou D-phénylalanyle, L ou D-homocystéyle, L ou D-aspartyle, L ou D-glutamyle, L ou D-hystidyle, L ou D-méthionyle, L ou D-thréonyle, L ou D-séryle, L ou D-cystéinyle, L ou D-leucyle, L ou D-arginyle, L ou D-tryptophanyle, L ou D-tyrosyle, L ou D-lysyle et L ou D-ornithyle.

Dans le cas ou $X^1$ = $X^4$ = Cys le dérivé peptidique peut être cyclisé par un pont difulfure en composé de formule

```
        X2  -  Gly  -  Asp  -  X3
        |                      |
        CO                     NH
        |                      |
  H2N-CH                       CH - COOH
        |                      |
        CH2  -  S   -   S  -  CH2
```

Les groupes N-protecteurs physiologiquement acceptables sont notamment les groupes protecteurs de l'attaque en N-terminal des enzymes exopeptidases. Comme exemples de tels groupes on peut citer les groupes acyles tels que les groupes t-butyloxycarbonyle (Boc), tert-amyl-oxycarbonyle (t-Aoc), benzyloxycarbonyle, benzoyle, acétyle, formyle, propanoyle, butanoyle, phénylacétyle, phenylpropanoyle, cyclo pentylcarbonyle.

La présente invention englobe également les équivalents des dérivés peptidiques de formule I dans lesquels chaque liaison peptidique (-CO-NH-) entre deux résidus d'acide aminé de la formule générale I est remplacée par les structures suivantes :
- $CO-N(CH_3)-$ ; $-CO-O-$ ; $-CH_2-NH-$ ; $-CS-NH-$ ;
- $CO-CH_2$ ; $CH_2-S-$ ; $-CHOH-CH_2-$ ; $-HN-CO-$ ;
- $CH=CH-$ ; $-CH_2-CH_2-$.

ou dans lesquels le squelette peptidique présente un ou plusieurs groupes intercalés tels que des groupes $-CH_2-$, $-NH-$, $-O-$.

Des dérivés peptidiques préférés sont des peptides de formule

H - Arg - Gly - Asp - Trp - OH
et H - Arg - Gly - Asp - Phe - OH

La présente invention a également pour objet :
- une composition pharmaceutique comprenant à titre de principe actif un dérivé peptidique de formule I
- un agent de diagnostic comprenant un dérivé de peptidique de formule I

Les dérivés peptidiques de formule I peuvent être préparés de manière classique par synthèse peptidique en phase liquide ou solide par couplages successifs des différents résidus d'acides aminés à incorporer (de l'extrémité N-terminale vers l'extrémité C-terminale en phase liquide, ou de l'extrémité C-terminale vers l'extrémité N-terminale en phase solide) et dont les extrémités N-terminales et les chaînes latérales réactives sont préalablement bloquées par des groupements tels que ceux mentionnés ci-dessous :

1) Extrémité N-terminale protégée par : Boc

Bpoc

Fmoc

```
2)                         Groupement bloquant
           Résidu          la chaîne latérale
     - - - - - - - - - - - - - - - - - - - - - - - - - - - - - - - - - - - -
     Alanyle          H
     Arginyle         tosyle
     Asparagyle       H,xanthyle
     Aspartyle        O-benzyle
     Cystéyle         acétamidométhyle(Acm), 4-méthylbenzyle
                      (Meb), 4-méthoxybenzyle(Mob), S-ben-
                      zyle
     Glutamyle        O-benzyle
     Glutaminyle      xanthyle
     Glycyle          H
     Histydyle        tosyle, 2-4-dinitrophényle(Dnp)
     Isoleucyle       H
     Leucyle          H
     Lysyle           2-chlorobenzyloxycarbonyle(Clz), tri-
                      fluoroacétyle(TFA), formyle(For), ben-
                      zyloxycarbonyle(Z)
     Méthionyle       H
     Norleucyle       H
     Ornithyle        benzyloxycarbonyle
     Phénylalanyle    H
     Propyle          H
     Pyroglutamyle    H
     Sarcosyle        H
     Séryle           O-benzyle
     Thréonyle        H, O-benzyle
     Tryptophanyle    H, formyle
     Tyrosyle         H, 2-6-dichlorobenzyle(dcb), 2-bromo-
                      benzyloxycarbonyle
     Valyle           H
```

On peut utiliser différentes méthodes de couplage :

1. Couplage des résidus par un carbodiimide (ex : DCC, EDC) avec ou sans catalyse (ex : HOBT) ou autre agent couplant (ex : EEDQ)

2. Utilisation des acides aminés sous forme d'anhydrides symétriques préformés

3. Utilisation des acides aminés sous forme d'esters activés (ex : p-nitrophénylester, HOBT ester) et couplage par l'intermédiaire de DCC.

En synthèse en phase solide (SPPS) le tableau I ci-dessous mentionne les différents types de résine utilisable ainsi que les protections et méthodes adéquates pour les différentes étapes.

## Tableau I

| Système SPPS utilisé | Liaison peptide-résine | Protection en alpha | réactif de déprotection | protection chaîne latérale | réactif de clivage |
|---|---|---|---|---|---|
| Classique | Benzyl ester | Boc | TFA, HCl | Benzyl | HF, HBr |
| Stable (longue chaîne) | Pam | Boc | TFA, HCl | Benzyl | HF, HBr |
| | Benzyl ester | Bpoc | TFA dilué | Benzyl | HF |
| | Benzyl ester | Bpoc | TFA dilué | t-Butyl | HF |
| Labile | Ether résine | Bpoc | TFA dilué | t-Butyl | TFA |
| Orthogonal | Ether résine | Fmoc | Piperidine | t-Butyl | TFA |
| Synthèse de segment | Ether résine | Fmoc | Piperidine | Benzyl | TFA |
| | t-Butyl résine | Fmoc | Piperidine | Benzyl | TFA |
| | Hydrazide résine | Fmoc | Piperidine | Benzyl | TFA |
| Assemblement de segments | Benzyl ester | Fmoc | Piperidine | Benzyl | HF |
| Peptides amides | MBHA, BHA | Boc | TFA, HCl | Benzyl | HF |
| Peptides alcools | Benzyl ester | Boc | TFA, HCl | Benzyl | $LiBH_4$ |

En synthèse en phase solide le 1er acide aminé (extrémité C-terminale) à fixer sur la résine peut être soit acquis commercialement déjà attaché au support soit fixé par l'intermédiaire de sel de césium (méthode de Gisin), d'un sel de tétraméthylammonium (méthode de Loffet) ou d'un carbodiimide.

Les exemples suivants illustrent la préparation des dérivés peptidiques de formule I

Exemple 1 :

Synthèse du peptide

H-Arg-Gly-Asp-Trp-OH (ou R G D W)

Ce peptide a été synthétisé en phase solide à partir d'une résine de type PAM (4-(oxyméthyl)-phénylacétamidométhyl) présubstituée Boc-Trp(CHO)-PAM-résine dont la substitution est de 0,34 mmol Trp/g. Les acides aminés protégés utilisés et leur milieu de solubilisation ont été les suivants :

| Dérivés | Solvants |
|---|---|
| Boc-Asp (O Bzl) | DMC |
| Boc-Gly | DMC |
| Boc-Arg (Tos) | DMF |

Chaque couplage a été réalisé en 2 heures par le dicyclohexylcarbodiimide (DCC) catalysé par l'hydroxy benzotriazole (HOBT) et suivi par une acétylation de 30 minutes par l'anhydride acétique.

En cours de synthèse les déprotections par l'acide trifluoroacétique (TFA) et les couplages ont été contrôlés par un test de Kaiser.

Le clivage final a été réalisé par HF (10 ml HF/g) en présence d'éthanedithiol (1 ml /10 ml HF) pour protéger le tryptophane pendant 1 heure à 0°C. Après lavage à l'éther, le peptide a été extrait par de l'acide acétique 45% puis 10% et lyophilisé.

5

Cependant le groupement formyl (-CHO) utilisé comme protecteur du Trp pendant la synthèse est résistant à l'acide fluorhydrique (HF). Pour l'éliminer un 2ème traitement du peptide est nécessaire :

A cet effet on traite le peptide RGDW (For) par 75 ml d'hydroxylamine 0,03 M ($H_2$N-OH,HCl) basifiée à pH 9 par de l'ammoniaque pendant plusieurs heures, en suivant par spectre d'absorption optique la disparition du groupement formyl à 300 nm et l'apparition du Tryptophane libre à 280 nm.

Après filtration sur Millex SR-0,5 $\mu$, le peptide obtenu a été purifié sur colonne de Sephadex G 10 éluée par de l'acide acétique à 25% puis lyophilisé 2 fois.

L'analyse d'acides aminés et le spectre d'absorption du RGDW montrent, ainsi que les chromatographies en couche mince et l'HPLC, un produit pur au point de vue peptidique et organique, mais révélant la présence d'une grande quantité de sel. Ceci s'explique par le traitement préalable de déprotection du Tryptophane par $H_2$N-OH,HCl en présence de $NH_4$OH qui entraîne la formation de chlorure d'ammonium non lyophilisable. Les chlorures ont donc dû être retenus par échange d'anion sur une colonne AG1-$X_2$ - (forme acétate) et le peptide élué par de l'acide acétique à 0,1% (pH 3,5). L'acétate d'ammonium obtenu est lyophilisable.

Contrôle de pureté

1 . Chromatographie sur couche mince de silice avec détection par :
- ninhydrine ($NH_2$)
- TDM (NH)
- réactif d'Ehrlich (paradiméthylaminobenzaldéhyde) (spécifique du Trp)

| Solvants de migration | Rf. |
|---|---|
| Butanol-1/Acide Acétique/Eau<br>4      1      1 | 0,1 |
| Méthanol/chloroforme/ammoniaque 25%<br>60      40      20 | 0,42 |

2.HPLC sur colonne analytique Spherisorb OD5.2 5 $\mu$
Eluant : gradient d'Acétonitrile (TFA 0.1%) dans $H_2$O (TFA 0.1%), de 0 à 80% en 25 nm, à 1 ml/nm.
Détection : Densité optique à 205-215 nm (liaison peptidique) et à 270-290 nm (Trp)
T Rétention RGDW : 15 nm soit 48% d'acétonitrile (TFA 0,1%).
3. Analyse d'acides aminés
Après hydrolyse à 110°C pendant 27 heures par HCl/Acide propionique (50/50) en présence de mercaptoéthanol (0,1%).

| Equivalent molaire | |
|---|---|
| Trp | ND |
| Asp | 1 |
| Cly | 0,92 |
| Arg | 0,95 |

Exemple 2 :

Synthèse du peptide

H-Arg-Gly-Asp-Phe-OH (ou R G D F)

Ce peptide a été synthétisé en phase solide en utilisant les méthodes suivantes :
- support : résine Merrifield chlorométhylée (0.7 mmol Cl/g) à 2% de divinylbenzène.
  Préparation de Boc-Phe-résine :
  7,5 mmoles (2g) de Boc-Phe-OH solubilisées dans 10 ml d'éthanol ont été additionnées de 3,75 mmoles (1,22g) de $Cs_2CO_3$ dans 2 ml d'eau. Le sel de césium formé (Boc-Phe-OCs, 7,5 mmol) après quelques minutes d'agitation a été évaporé à sec et mis au dessicateur sous vide pendant 48 h. Il a été ensuite resolubilisé par 80 ml de DMF et mélangé à 10,7g de résine (0.7 mmol Cl). La réaction a été réalisée en 24 h. sous agitation dans un bain chauffant à 50°C. La résine dérivatisée (Boc-Phe-résine) a été ensuite filtrée et lavée par DMF, DFM/$H_2O$, DMF, EtOH, puis séché sous vide pendant 3 h. Le taux de substitution de la résine a été calculé par analyse d'acide aminé après hydrolyse à 150°C, pendant 3 h., par le mélange HCl/acide propionique (50/50). La substitution obtenue a été de 0,24 mmol Phe/g résine.
- couplage :
  Tous les dérivés aminés incorporés ont été couplés par la méthode du carbodiimide DCC en présence de catalyseur HOBT (2 équivalents Phe en acide aminé, en DCC et en HOBT).
  Les dérivés protégés utilisés et leur milieu de solubilisation ont été les suivants :

| Dérivés | Solvants |
|---|---|
| Boc-Asp-O-Bzl | DCM |
| Boc-Gly | DCM |
| Boc-Arg(Tos) | DMF |

Les couplages ont été réalisés en 2 h. dans du DCM.
Les déprotections par TFA et les couplages ont tous été contrôlés par un test de Kaiser.
- clivage :
  Le clivage du peptide synthétisé a été effectué par HF (10 ml/g résine) en présence d'anisol (1 mg/g rés.) pendant 1 h. à 0°C. Après lavage à l'éther, le peptide à été ensuite extrait par de l'acide acétique à 15% et lyophilisé.
- purification : sur colonne de Sephadex G10 éluée par de l'acide acétique à 10%
- contrôle :
  . analyse d'acide aminé : après hydrolyse pendant 30 mn à 150°C par HCl/acide propionique 50/50
  . chromatographie sur couche mince de silice :
  éluant : $CH_3OH/CHCl_3/NH_4OH$ 25% (60/40/20)
  détection : phénantrène quinone (Arg)
  ninhydrine ($NH_2$)
  TDM (NH)
  $R_f = 0,51$
  éluant 2 : 2-butanone/$CH_3COOH/H_2O$ (10/30/25)
  $R_f = 0,73$
  éluant 3 : butanol/$CH_3COOH/H_2O$ (75/10/24)
  $R_f = 0,16$

Exemple 3 :

Synthèse du peptide :

H-Leu-Arg-Gly-Asp-Phe-OH (ou L R G D F).

Ce peptide a été synthétisé, purifié et analysé selon les mêmes méthodes que le peptide de l'exemple 1.
Ses caractéristiques analytiques sont les suivantes :
  . chromatographie sur couche mince de silice :
  éluant 1 :$CH_3OH/CHCl_3/NH_4OH$ 25% (60/40/20)
  $R_f = 0,82$

EP 0 275 748 B1

éluant 2 : 2-butanone/$CH_3COOH$/$H_2O$ (10/30/25)
$R_f$ = 0,69
éluant 3 : butanol/$CH_3COOH$/$H_2O$ (75/10/24)
$R_f$ = 0,21

On donnera ci-après des résultats des études pharmacologiques mettant en évidence les propriétés des dérivés peptidiques de formule I

## 1. Inhibition de la liaison plaquette-fibrinogène

Préparation des plaquettes

Les plaquettes sont isolées à partir de 60 ml de sang humain prélevé sur un tampon anticoagulant, l'ACD, à raison d'1 volume d'ACD pour 6 volumes de sang.
L'ACD a la composition suivante :

| Citrate trisodique 5 $H_2O$ | 5,95 g |
|---|---|
| Acide citrique | 3,41 g |
| Dextrose | 5 g |
| $H_2O$ | qsp 250 ml |

Le sang est ensuite centrifugé 20 mn à 1000 t/mn (centrifugeuse JOUAN E 96) à température ambiante.

Le PRP (plasma riche en plaquettes) est décanté et additionné de $PGE_1$ 0,1 $\mu$M puis centrifugé 15 mn à 2000 t/mn.

Les plaquettes obtenues dans le culot sont alors reprises par 1 ml de tampon Tyrode-albumine pH 7,2 préparé selon la composition suivante :

| Tampon Tyrode (solution mère) : | |
|---|---|
| NaCl | 1,3 M |
| KCl | 0,026 M |
| $NaHCO_3$ | 0,12 M |

| Tampon Tyrode-albumine : | |
|---|---|
| solution mère | 1/10 M |
| D-glucose | 0,0055 M |
| albumine | 2% |
| HCl 1M | qsp pH 7,2 |

Les plaquettes sont lavées sur une colonne de Sépharose CL 2B par du tampon Tyrode-albumine pH 7,2 puis les plaquettes recueillies sont diluées à la concentration de $2.10^8$ pl./ml.

Les essais sont réalisés sur $4.10^7$ plaquettes en présence de $CaCl_2$ (0,5 mM), de $^{125}$I-fibrinogène($0,1.10^{-6}$ M) et de différentes concentrations de peptide, et la stimulation des plaquettes est provoquée par de l'ADP ($5.10^{-6}$ M). Après 15 mn d'incubation et dépôt sur une solution de saccharose à 15% le complexe $^{125}$I-fibrinogène-plaquette est isolé par centrifugation à 12000 t/mn pendant 2 mn.

Les résultats sont donnés sous forme de CI50 dans le tableau II. Ce tableau donne également la séquence des peptides.

## 2. Inhibition de l'agrégation plaquettaire

L'effet des peptides synthétiques sur l'agrégation plaquettaire a été étudié sur des plaquettes isolées comme précédemment pour l'étude de la liaison au fibrinogène. La stimulation des plaquettes est également obtenue par l'ADP $5.10^{-6}$ M et l'essai réalisé en présence de fibrinogène $1,1.10^{-6}$ M et de $CaCl_2$ $0,5.10^{-6}$ M.

8

EP 0 275 748 B1

Les résultats sont donnés dans le tableau II et mettent en évidence une activité inhibitrice sur l'agrégation plaquettaire des peptides de formule I.

## Tableau II

| Peptide | Nomenclature | Inhibition IC50 (µM) | |
|---|---|---|---|
| | | Liaison plaquettaire Fibrinogène | Agrégation |
| Arg - Gly - Asp - Trp | R G D W | 3,6 | 10 |
| Arg - Gly - Asp - Phe | R G D F | 7 | 15 |
| Leu - Arg - Gly - Asp - Phe | L R G D F | 20 | 20 |

Les dérivés peptidiques de formule I peuvent être utilisés notamment pour le traitement et la prévention des thromboses, en particulier dans les états préthrombotiques pour bloquer l'agrégation plaquettaire.

Ils peuvent également exercer un effet inhibiteur sur
- l'adhésion des plaquettes sanguines aux cellules endothéliales des parois vasculaires ou du sous endothélium.
- l'athérogénèse
- le développement de métastases
- la réponse inflammatoire

Les compositions thérapeutiques selon l'invention peuvent être administrées à l'homme ou aux animaux par voie orale ou parentérale.

Elles peuvent être sous la forme de préparation solides, semi-solides ou liquides. Comme exemples, on peut citer les comprimés, les gélules, les solutions ou les suspensions injectables.

Dans ces compositions le principe actif est généralement mélangé avec un ou plusieurs excipients pharmaceutiquement acceptables habituels bien connus de l'homme de l'art.

Les compositions thérapeutiques peuvent contenir notamment de 1 à 60% en poids de principe actif.

La quantité de principe actif administrée dépend évidemment du patient qui est traité, de la voie d'administration et de la sévérité de la maladie. Elle est généralement de 1 à 5000 mg.

ANNEXE

Abréviations usuelles de la chimie des peptides

BHA : benzylhydrylamine (résine)
Boc : tert-butyloxycarbonyle
Bpoc : 2-(4-biphénylyl)propyl(-2)oxycarbonyle
Bzl : benzyle
Clz : 2-chlorobenzyloxycarbonyle
DCC : dicyclohexylcarbodiimide
DCM : dichlorométhane
DMF : diméthylformamide
EDC : N-ethyl-N'-(3-diméthylaminopropyl) carbodiimide
EEDQ : N-ethyloxycarbonyl-2-ethyloxy-1,2-dihydroquinoline
Fmoc : 9-fluorénylméthyloxycarbonyle
HOBT : 1-hydroxybenzotriazole
MBHA : 4-méthylbenzhydrylamine (résine)
TDM : N,N,N',N' ,tétraméthyl-4,4'-diaminodiphénylméthane
TFA : acide trifluoroacétique
Tos : tosyle
Xan : xanthyle

9

EP 0 275 748 B1

| Symboles des acides aminés | | |
|---|---|---|
| A | Ala | alanine |
| C | Cys | cystéine |
| D | Asp | acide aspartique |
| E | Glu | acide glutamique |
| F | Phe | phénylalanine |
| G | Gly | glycine |
| H | His | histidine |
| I | Ile | isoleucine |
| K | Lys | lysine |
| L | Leu | leucine |
| M | Met | méthionine |
| N | Asn | asparagine |
| P | Pro | proline |
| Q | Gln | glutamine |
| R | Arg | arginine |
| S | Ser | serine |
| T | Thr | thréonine |
| V | Val | valine |
| W | Trp | tryptophane |
| Y | Tyr | tyrosine |

**Revendications**

**Revendications pour les Etats contractants suivants : AT, BE, CH, DE, FR, GB, IT, LI, LU, NL, SE**

**1.** Dérivés peptidiques de formule

$$X^1 - X^2 - Gly - Asp - X^3 - X^4 \qquad (I)$$

dans laquelle

$X^1$ représente l'hydrogène, un groupe N-protecteur, un résidu d'acide aminé ou un résidu d'acide aminé N- protégé,

$X^2$ représente un résidu L-Arg ou D-Arg, L-Orn ou D-Orn, N-aminocarbonyl L-Orn ou N-amino carbonyl-D-Orn, ou L-Lys ou D-Lys,

$X^3$ représente un résidu L-Trp,N-Trp,L-Leu, D-Leu,L-Ile,D-Ile,L-Phe,D-Phe ou un enchaînement de 2 ou 3 de ces résidus, et

$X^4$ représente un groupe -OH, -NH$_2$, -OR$^1$ avec R$^1$ représentant un radical alkyle en C$_1$ à C$_4$, NHR$^2$ avec R$^2$ représentant un radical alkyle en C$_1$ à C$_4$, ou un résidu d'acide aminé,

$X^1$ et $X^4$ pouvant former un pont disulfure lorsqu'ils représentent tous deux un résidu Cys.

**2.** Dérivés peptidiques selon la revendication 1 dans lesquels,

$X^1$ représente l'hydrogène ou un résidu L-Leu ou D-Leu,

$X^2$ représente un résidu L-Arg ou D-Arg,

$X^3$ représente un résidu L-Trp,D-Trp,L-Leu, D-Leu,L-Ile,D-Ile,L-Phe ou D-Phe, et

$X^4$ représente un groupe OH.

**3.** Peptide de formule

H - Arg - Gly - Asp - Trp - OH.

**4.** Peptide de formule

H - Arg - Gly - Asp - Phe - OH.

**5.** Peptide de formule

H - Leu - Arg - Gly - Asp - Phe - OH.

6. Composition thérapeutique, caractérisée en ce qu'elle contient à titre de principe actif un dérivé peptidique selon l'une quelconque des revendications 1 à 5.

7. Agent de diagnostic, caractérisé en ce qu'il contient un dérivé peptidique selon l'une quelconque des revendications 1 à 5.

**Revendications pour l'Etat contractant suivant : ES**

1. Procédé de préparation de dérivés peptidiques de formule

$X^1 - X^2$ - Gly - Asp - $X^3$ - $X^4$    (I)

dans laquelle
   $X^1$ représente l'hydrogène, un groupe N-protecteur, un résidu d'acide aminé ou un résidu d'acide aminé N- protégé,
   $X^2$ représente un résidu L-Arg ou D-Arg, L-Orn ou D-Orn, N-aminocarbonyl L-Orn ou N-amino carbonyl-D-Orn, ou L-Lys ou D-Lys,
   $X^3$ représente un résidu L-Trp,N-Trp,L-Leu, D-Leu,L-Ile,D-Ile,L-Phe,D-Phe ou un enchaînement de 2 ou 3 de ces résidus, et
   $X^4$ représente un groupe -OH, -$NH_2$, -$OR^1$ avec $R^1$ représentant un radical alkyle en $C_1$ à $C_4$, $NHR^2$ avec $R^2$ représentant un radical alkyle en $C_1$ à $C_4$, ou un résidu d'acide aminé,
   caractérisé en ce que l'on prépare ces dérivés par synthèse peptidique.

2. Procédé selon la revendication 1, caractérisé en ce que l'on prépare un dérivé peptidique de formule I dans laquelle,
   $X^1$ représente l'hydrogène ou un résidu L-Leu ou D-Leu,
   $X^2$ représente un résidu L-Arg ou D-Arg,
   $X^3$ représente un résidu L-Trp,D-Trp,L-Leu, D-Leu,L-Ile,D-Ile,L-Phe ou D-Phe, et
   $X^4$ représente un groupe OH.

3. Procédé selon la revendication 1, caractérisé en ce que l'on prépare un peptide de formule

H - Arg - Gly - Asp - Trp - OH.

4. Procédé selon la revendication 1, caractérisé en ce que l'on prépare un peptide de formule

H - Arg - Gly - Asp - Phe - OH.

5. Procédé selon la revendication 1, caractérisé en ce que l'on prépare un peptide de formule

H - Leu - Arg - Gly - Asp - Phe - OH.

6. Procédé de préparation d'une composition thérapeutique, caractérisé en ce que l'on met un dérivé peptidique tel que défini à la revendication 1 sous une forme pharmaceutiquement acceptable.

7. Utilisation d'un dérivé peptidique tel que défini à la revendication 1 pour la préparation d'une composition pharmaceutique.

8. Agent de diagnostic, caractérisé en ce qu'il contient un dérivé peptidique tel que défini à la revendication 1.

**Revendications pour l'Etat contractant suivant : GR**

1. Dérivés peptidiques de formule

$X^1 - X^2$ - Gly - Asp - $X^3$ - $X^4$    (I)

dans laquelle

X$^1$ représente l'hydrogène, un groupe N-protecteur, un résidu d'acide aminé ou un résidu d'acide aminé N- protégé,

X$^2$ représente un résidu L-Arg ou D-Arg, L-Orn ou D-Orn, N-aminocarbonyl L-Orn ou N-amino carbonyl-D-Orn, ou L-Lys ou D-Lys,

X$^3$ représente un résidu L-Trp,N-Trp,L-Leu, D-Leu,L-Ile,D-Ile,L-Phe,D-Phe ou un enchaînement de 2 ou 3 de ces résidus, et

X$^4$ représente un groupe -OH, -NH$_2$, -OR$^1$ avec R$^1$ représentant un radical alkyle en C$_1$ à C$_4$, NHR$^2$ avec R$^2$ représentant un radical alkyle en C$_1$ à C$_4$, ou un résidu d'acide aminé,

X$^1$ et X$^4$ pouvant former un pont disulfure lorsqu'ils représentent tous deux un résidu Cys.

2.  Dérivés peptidiques selon la revendication 1 dans lesquels,

X$^1$ représente l'hydrogène ou un résidu L-Leu ou D-Leu,

X$^2$ représente un résidu L-Arg ou D-Arg,

X$^3$ représente un résidu L-Trp,D-Trp,L-Leu, D-Leu,L-Ile,D-Ile,L-Phe ou D-Phe, et

X$^4$ représente un groupe OH.

3.  Peptide de formule

H - Arg - Gly - Asp - Trp - OH.

4.  Peptide de formule

H - Arg - Gly - Asp - Phe - OH.

5.  Peptide de formule

H - Leu - Arg - Gly - Asp - Phe - OH.

6.  Procédé de préparation d'une composition thérapeutique, caractérisé en ce que l'on met un dérivé peptidique selon l'une quelconque des revendications 1 à 5 sous une forme pharmaceutiquement acceptable.

7.  Utilisation d'un dérivé peptidique selon l'une quelconque des revendications 1 à 5 pour la préparation d'une composition pharmaceutique.

8.  Agent de diagnostic, caractérisé en ce qu'il contient un dérivé peptidique selon l'une quelconque des revendications 1 à 5.

**Claims**
**Claims for the following Contracting States : AT, BE, CH, DE, FR, GB, IT, LI, LU, NL, SE**

1.  Peptide derivatives of the formula

X$^1$ - X$^2$ - Gly - Asp - X$^3$ - X$^4$     (I)

in which

X$^1$ represents hydrogen, an N-protecting group, an amino acid residue or an N-protected amino acid residue,

X$^2$ represents a residue of L-Arg or D-Arg, L-Orn or D-Orn, N-aminocarbonyl-L-Orn or N-aminocarbonyl-D-Orn, or L-Lys or D-Lys,

X$^3$ represents a residue of L-Trp, N-Trp, L-Leu, D-Leu, L-Ile, D-Ile, L-Phe or D-Phe or a chain containing two or three of these residues, and

X$^4$ represents a -OH, -NH$_2$, -OR$^1$ group in which R$^1$ represents a C$_1$ to C$_4$ alkyl radical, an NHR$^2$ group in which R$^2$ represents a C$_1$ to C$_4$ alkyl radical, or an amino acid residue,

X$^1$ and X$^4$ being able to form a disulphide bridge when they both represent a Cys residue.

2. Peptide derivatives according to Claim 1, in which
$X^1$ represents hydrogen or an L-Leu or D-Leu residue,
$X^2$ represents an L-Arg or D-Arg residue,
$X^3$ represents a residue of L-Trp, D-Trp, L-Leu, D-Leu, L-Ile, D-Ile, L-Phe or D-Phe, and
$X^4$ represents an OH group.

3. A peptide of the formula

H - Arg - Gly - Asp - Trp - OH.

4. A peptide of the formula

H - Arg - Gly - Asp - Phe - OH.

5. A peptide of the formula

H - Leu - Arg - Gly - Asp - Phe - OH.

6. A therapeutic composition, characterised in that it contains, as an active principle, a peptide derivative according to any one of Claims 1 to 5.

7. A diagnostic agent, characterised in that it contains a peptide derivative according to any one of Claims 1 to 5.

**Claims for the following Contracting State : ES**

1. A method of preparing peptide derivatives of the formula

$X^1$ - $X^2$ - Gly - Asp - $X^3$ - $X^4$     (I)

in which
$X^1$ represents hydrogen, an N-protecting group, an amino acid residue or an N-protected amino acid residue,
$X^2$ represents a residue of L-Arg or D-Arg, L-Orn or D-Orn, N-aminocarbonyl-L-Orn or N-aminocarbonyl-D-Orn, or L-Lys or D-Lys,
$X^3$ represents a residue of L-Trp, N-Trp, L-Leu, D-Leu, L-Ile, D-Ile, L-Phe or D-Phe or a chain containing two or three of these residues, and,
$X^4$ represents a -OH, -$NH_2$, -$OR^1$ group in which $R^1$ represents a $C_1$ to $C_4$ alkyl radical, an $NHR^2$ group in which $R^2$ represents a $C_1$ to $C_4$ alkyl radical, or an amino acid residue,
characterised in that these derivatives are prepared by peptide synthesis.

2. A method according to Claim 1, characterised in that a peptide derivative of formula I is prepared, in which
$X^1$ represents hydrogen or an L-Leu or D-Leu residue,
$X^2$ represents an L-Arg or D-Arg residue,
$X^3$ represents a residue of L-Trp, D-Trp, L-Leu, D-Leu, L-Ile, D-Ile, L-Phe or D-Phe, and
$X^4$ represents an OH group.

3. A method according to Claim 1, characterised in that a peptide is prepared having the formula

H - Arg - Gly - Asp - Trp - OH.

4. A method according to Claim 1, characterised in that a peptide is prepared having the formula

H - Arg - Gly - Asp - Phe - OH.

5. A method according to Claim 1, characterised in that a peptide is prepared having the formula

H - Leu - Arg - Gly - Asp - Phe - OH.

6. A method of preparing a therapeutic composition, characterised in that a peptide derivative as defined in Claim 1 is put in a pharmaceutically acceptable form.

7. The use of a peptide derivative as defined in Claim 1 for the preparation of a pharmaceutical composition.

8. A diagnostic agent, characterised in that it contains a peptide derivative as defined in Claim 1.

**Claims for the following Contracting State : GR**

1. Peptide derivatives of the formula

$X^1$ - $X^2$ - Gly - Asp - $X^3$ - $X^4$ (I)

in which
$X^1$ represents hydrogen, an N-protecting group, an amino acid residue or an N-protected amino acid residue,
$X^2$ represents a residue of L-Arg or D-Arg, L-Orn or D-Orn, N-aminocarbonyl-L-Orn or N-aminocarbonyl-D-Orn, or L-Lys or D-Lys,
$X^3$ represents a residue of L-Trp, N-Trp, L-Leu, D-Leu, L-Ile, D-Ile, L-Phe or D-Phe or a chain containing two or three of these residues, and
$X^4$ represents a -OH, -$NH_2$, -$OR^1$ group in which $R^1$ represents a $C_1$ to $C_4$ alkyl radical, an $NHR^2$ group in which $R^2$ represents a $C_1$ to $C_4$ alkyl radical, or an amino acid residue,
$X^1$ and $X^4$ being able to form a disulphide bridge when they both represent a Cys residue.

2. Peptide derivatives according to Claim 1, in which
$X^1$ represents hydrogen or an L-Leu or D-Leu residue,
$X^2$ represents an L-Arg or D-Arg residue,
$X^3$ represents a residue of L-Trp, D-Trp, L-Leu, D-Leu, L-Ile, D-Ile, L-Phe or D-Phe, and
$X^4$ represents an OH group.

3. A peptide of the formula

H - Arg - Gly - Asp - Trp - OH.

4. A peptide of the formula

H - Arg - Gly - Asp - Phe - OH.

5. A peptide of the formula

H - Leu - Arg - Gly - Asp - Phe - OH.

6. A method of preparing a therapeutic composition, characterised in that a peptide derivative according to any one of Claims 1 to 5 is put in a pharmaceutically acceptable form.

7. The use of a peptide derivative according to any one of Claims 1 to 5 for the preparation of a pharmaceutical composition.

8. A diagnostic agent, characterised in that it contains a peptide derivative according to any one of Claims 1 to 5.

**Patentansprüche**
**Patentansprüche für folgende Vertragsstaaten : AT, BE, CH, DE, FR, GB, IT, LI, LU, NL, SE**

1. Peptidderivate der Formel

$X^1$ - $X^2$ - Gly - Asp - $X^3$ - $X^4$      (I),

in der

$X^1$ für Wasserstoff, eine N-Schutzgruppe, ein Aminosäurerest oder ein N-geschützter Aminosäurerest,

$X^2$ für einen L-Arg- oder D-Arg-, L-Orn- oder D-Orn-, N-Aminocarbonyl- L-Orn- oder N-Aminocarbonyl-D-Orn- oder L-Lys- oder D-Lys-Rest,

$X^3$ für einen L-Trp-, N-Trp-, L-Leu-, D-Leu-, L-Ile-, D-Ile-, L-Phe-, D-Phe-Rest oder eine Kette von 2 oder 3 dieser Reste, und

$X^4$ für eine OH-Gruppe, $NH_2$-Gruppe, $OR^1$-Gruppe, stehen, wobei $R^1$ für eine Alkylgruppe mit 1 bis 4 Kohlenstoffatomen, $NHR^2$ mit $R^2$ für eine Alkylgruppe mit 1 bis 4 Kohlenstoffatomen oder einen Aminosäurerest steht, und wobei

$X^1$ und $X^4$ eine Disulfurbrücke bilden können, wenn sie beide der Rest Cys sind.

2. Peptidderivate nach Anspruch 1, wobei

$X^1$ für Wasserstoff oder einen L-Leu- oder D-Leu-Rest,

$X^2$ für einen L-Arg- oder D-Arg-Rest,

$X^3$ für einen L-Trp-, D-Trp-, L-Leu-, D-Leu-, L-Ile-, D-Ile-, L-Phe- oder D-Phe-, und

$X^4$ für den OH-Rest stehen.

3. Peptid der Formel

H - Arg - Gly - Asp - Trp -OH.

4. Peptid der Formel

H - Arg - Gly - Asp - Phe - OH.

5. Peptid der Formel

H - Leu - Arg - Gly - Asp - Phe - OH.

6. Therapeutische Zusammensetzung, gekennzeichnet durch Gehalt an einem Peptidderivat gemäß einem der Ansprüche 1 bis 5 als Wirkstoff.

7. Diagnostisches Mittel, gekennzeichnet durch Gehalt eines Peptidderivats nach einem der Ansprüche 1 bis 5.

**Patentansprüche für folgenden Vertragsstaat : ES**

1. Verfahren zur Herstellung von Peptidderivaten der Formel

$X^1$ - $X^2$ - Gly - Asp - $X^3$ - $X^4$      (I),

in der

$X^1$ für Wasserstoff, eine N-Schutzgruppe, ein Aminosäurerest oder ein N-geschützter Aminosäurerest,

$X^2$ für einen L-Arg- oder D-Arg-, L-Orn- oder D-Orn-, N-Aminocarbonyl- L-Orn- oder N-Aminocarbonyl-D-Orn- oder L-Lys- oder D-Lys-Rest,

$X^3$ für einen L-Trp-, N-Trp-, L-Leu-, D-Leu-, L-Ile-, D-Ile-, L-Phe-, D-Phe-Rest oder eine Kette von 2 oder 3 dieser Reste, und

$X^4$ für eine OH-Gruppe, $NH_2$-Gruppe, $OR^1$-Gruppe, stehen, wobei $R^1$ für eine Alkylgruppe mit 1 bis 4 Kohlenstoffatomen, $NHR^2$ mit $R^2$ für eine Alkylgruppe mit 1 bis 4 Kohlenstoffatomen oder einen Aminosäurerest steht,

dadurch gekennzeichnet, daß man diese Derivate durch Peptidsynthese herstellt.

2. Verfahren nach Anspruch 1, dadurch gekennzeichnet, daß man ein Peptidderivat der Formel I herstellt,

in der

X$^1$ für Wasserstoff oder einen L-Leu- oder D-Leu-Rest,

X$^2$ für einen L-Arg- oder D-Arg-Rest,

X$^3$ für einen L-Trp-, D-Trp-, L-Leu-, D-Leu-, L-Ile-, D-Ile-, L-Phe- oder D-Phe-, und

X$^4$ für den OH-Rest stehen.

**3.** Verfahren zur Herstellung nach Anspruch 1, dadurch gekennzeichnet, daß man ein Peptid der Formel

H - Arg - Gly - Asp - Trp -OH herstellt.

**4.** Verfahren nach Anspruch 1, dadurch gekennzeichnet, daß man ein Peptid der Formel

H - Arg - Gly - Asp - Phe - OH herstellt.

**5.** Verfahren nach Anspruch 1, dadurch gekennzeichnet, daß man ein Peptid der Formel

H - Leu - Arg - Gly - Asp - Phe - OH herstellt.

**6.** Verfahren zur Herstellung einer therapeutischen Zusammensetzung, dadurch gekennzeichnet, daß man ein Peptidderivat nach Anspruch 1 in eine pharmazeutisch verträgliche Form bringt.

**7.** Verwendung eines Peptidderivats nach Anspruch 1 für die Herstellung einer pharmazeutischen Zusammensetzung.

**8.** Diagnosemittel, gekennzeichnet durch Gehalt an einem Peptidderivat nach Anspruch 1.

**Patentansprüche für folgenden Vertragsstaat : GR**

**1.** Peptidderivate der Formel

X$^1$ - X$^2$ - Gly - Asp - X$^3$ - X$^4$        (I),

in der

X$^1$ für Wasserstoff, eine N-Schutzgruppe, ein Aminosäurerest oder ein N-geschützter Aminosäurerest,

X$^2$ für einen L-Arg- oder D-Arg-, L-Orn- oder D-Orn-, N-Aminocarbonyl- L-Orn- oder N-Aminocarbonyl-D-Orn- oder L-Lys- oder D-Lys-Rest,

X$^3$ für einen L-Trp-, N-Trp-, L-Leu-, D-Leu-, L-Ile-, D-Ile-, L-Phe-, D-Phe-Rest oder eine Kette von 2 oder 3 dieser Reste, und

X$^4$ für eine OH-Gruppe, NH$_2$-Gruppe, OR$^1$-Gruppe, stehen, wobei R$^1$ für eine Alkylgruppe mit 1 bis 4 Kohlenstoffatomen, NHR$^2$ mit R$^2$ für eine Alkylgruppe mit 1 bis 4 Kohlenstoffatomen oder einen Aminosäurerest steht, und wobei

X$^1$ und X$^4$ eine Disulfurbrücke bilden können, wenn sie beide der Rest Cys sind.

**2.** Peptidderivate nach Anspruch 1, wobei

X$^1$ für Wasserstoff oder einen L-Leu- oder D-Leu-Rest,

X$^2$ für einen L-Arg- oder D-Arg-Rest,

X$^3$ für einen L-Trp-, D-Trp-, L-Leu-, D-Leu-, L-Ile-, D-Ile-, L-Phe- oder D-Phe-, und

X$^4$ für den OH-Rest stehen.

**3.** Peptid der Formel

H - Arg - Gly - Asp - Trp -OH.

**4.** Peptid der Formel

H - Arg - Gly - Asp - Phe - OH.

**5.** Peptid der Formel

H - Leu - Arg - Gly - Asp - Phe - OH.

**6.** Verfahren zur Herstellung einer therapeutischen Zusammensetzung, dadurch gekennzeichnet, daß man ein Peptidderivat nach einem der Ansprüche 1 bis 5 in pharmazeutisch verträgliche Form bringt.

**7.** Verwendung eines Peptidderivats gemäß einem der Ansprüche 1 bis 5 für die Herstellung einer pharmazeutischen Zusammensetzung.

**8.** Diagnosemittel, gekennzeichnet durch Gehalt an einem Peptidderivat nach einem der Ansprüche 1 bis 5.